Europäisches Patentamt

European Patent Office

Office européen des brevets

(11) Publication number: **0 371 783 B1**

(12) **EUROPEAN PATENT SPECIFICATION**

(45) Date of publication of patent specification: **06.04.94**  (51) Int. Cl.⁵: **C12M 3/00**

(21) Application number: **89312421.4**

(22) Date of filing: **29.11.89**

(54) **Culturing method, system and apparatus for cell culture.**

(30) Priority: **30.11.88 JP 300968/88**

(43) Date of publication of application:
**06.06.90 Bulletin 90/23**

(45) Publication of the grant of the patent:
**06.04.94 Bulletin 94/14**

(84) Designated Contracting States:
**CH DE LI**

(56) References cited:
**EP-A- 224 734
EP-A- 336 966
WO-A-86/07605**

**HANDBOOK OF MEMBRANE TECHNOLOGY,
ed. H. Ohya, publ. by Saimai-shobau Book
Co. 715-717: Millipore Catalog & Purchasing
Guide (JP); p. 3&NUM;**

(73) Proprietor: **HITACHI, LTD.
6, Kanda Surugadai 4-chome
Chiyoda-ku, Tokyo 100(JP)**

(72) Inventor: **Ishida, Masahiko
20-8 Hanayama-cho 1-chome
Hitachi-shi Ibaraki-ken(JP)**
Inventor: **Haga, Ryoichi
11-3 Daihara-cho 3-chome
Hitachi-shi Ibaraki-ken(JP)**
Inventor: **Matsuzaki, Harumi
18-5 Daihara-cho 2-chome
Hitachi-shi Ibaraki-ken(JP)**

(74) Representative: **Paget, Hugh Charles Edward
et al
MEWBURN ELLIS
2 Cursitor Street
London EC4A 1BO (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

This invention relates to methods and apparatus for culturing living cells, and in particular embodiments to methods for a large scale, high concentration culturing, a culturing system and a culturing apparatus of living cells which performs suspension culture of living cells in a liquid medium and removes waste components efficiently.

Recently the production of monoclonal antibodies and interferon by the culture of animal cells, and the production of natural pigments by the culture of vegetable cells, have joined the known culture of microorganism cells. To produce such useful materials on an industrial basis so-called "high density culture" is necessary, by which cells can be cultured on a large scale for a long period of time while keeping them stably at a high concentration.

Nutrient components are consumed while waste components (such as lactate and the like) are generated in a culture tank during the cell propagation process. If this condition is allowed to persist, propagation of living cells is impeded and high density culture is not attained. Therefore, so-called "perfusion culture" is essential, which separates cells from the culture broth and removes cell-free waste solution to the outside of the system, exchanging it for fresh medium.

Since the cell is a kind of particle which has a specific gravity greater than that of the medium, perfusion culturing methods apply heretofore known solid-liquid separation techniques. As the cell separation methods, sedimentation separation, centrifugal separation and membrane filtration are ordinarily employed. A membrane filtration device is disposed in a culture tank of a culturing apparatus, or outside the culturing apparatus. This is described, for example in Scientific American 248 (1) p. 24 (1983) and in JP-B-102187/1985.

Our EP-A-336966, which is in Article 54(3) EPC relationship with the present case, describes culturing methods in which culture solution is filtered out of the culture tank via a membrane filter which is periodically back-washed with solution to reduce clogging. A debubbling layer is used in the culture tank to destroy foam caused by gases ($O_2$ and $CO_2$) blown into the culture tank for optimum culture conditions. Filtration may be in or away from the main culture tank.

Studies of conventional methods by the present inventors found that if a membrane filtration device is disposed in the culturing apparatus, cells attaching to the membrane surface propagate and bring about irreversible clogging. Once culture has started, exchange of the filtration device is not possible during culturing. Also, since the structure and position of the filtration device greatly affect mixing of the solutions in the culturing apparatus, and the supply of oxygen, scale-up of the apparatus is difficult to attain.

The latter two problems can be solved by disposing the filtration device outside the main culturing tank, but we find that if the filtration device is disposed outside the culturing apparatus and simple back-wash is carried out, it is still difficult to conduct perfusion-culture for a long period of time without clogging the film, particularly at a high concentration of $1 \times 10^7$ cells/ml or more.

It is desirable to provide novel culturing methods and apparatus for cell culture, which preferably eliminates damage to living cells and can avoid contamination of culture broth due to bacteria.

In one aspect, the invention provides a method of culturing living cells wherein cells are cultured in a cell culture broth in a culture chamber, the method comprising:

withdrawing cell culture broth from the culture chamber to a filtration device having a microfiltration membrane;

microfiltering the cell culture broth through the microfiltration membrane, while contacting the immersed surface of the membrane with a gas which is non-toxic to the cells;

using a hydrophobic defoaming layer at the liquid surface to rupture bubbles and thereby inhibit the formation of foam;

returning cells to the culture chamber after the microfiltration, and withdrawing filtrate from the culture.

In another aspect, the invention provides apparatus for cell culture, comprising;

(a) a culture vessel for holding a culture broth in which cells are cultured in a suspended state;

(b) a filtration device comprising a microfiltration membrane;

(c) means for transferring culture broth from the culturing vessel to the filtration device;

(d) means for blowing a gas into culture broth in the filtration device, to contact the microfiltration membrane;

(e) means for inhibiting foam formation on the culture broth surface;

(f) means for returning cells from the filtration device to the culture vessel, and

(g) means for withdrawing filtrate from the filtration device.

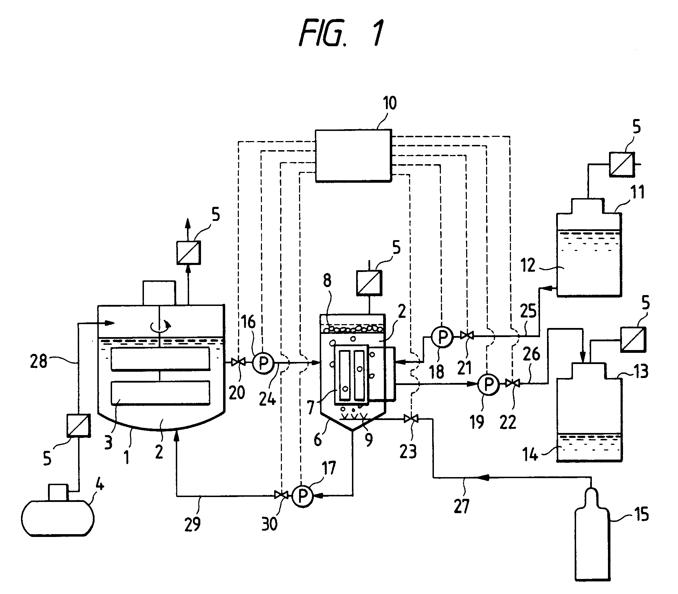

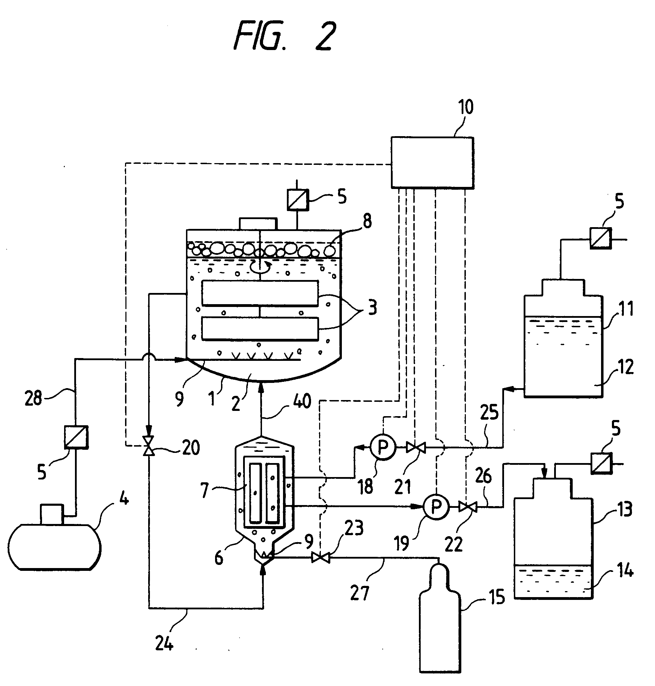

Figs. 1 and 2 are schematic views of culturing systems in accordance with embodiments of the present invention.

2

A feature of the present process resides in that a cell culture broth in a culturing apparatus is withdrawn to the outside of the apparatus, the withdrawn culture broth is subjected to filtration using a filtration membrane while a gas which is non-toxic to cells is in contact with the membrane under a reduced pressure so that the cells are separated from the liquid and returned into the culturing apparatus. Since filtration is made while the gas is in contact with the membrane surface, clogging of the filtration membrane due to cell propagation on the membrane surface is minimized. If the gas is not brought into contact with the membrane surface, the cells adhering to the membrane surface propagate and clog the pores of the membrane so that pressure loss increases drastically and filtration becomes impossible.

Protein-containing foams which are difficult to break down, and are formed on the liquid surface by the gas blown into the liquid, are effectively ruptured by a defoaming layer made of a hydrophobic material so that the filtration process can be continued. In addition to the proteins such as serum and albumin contained in the medium, the culture broth contains proteins which are excreted by the cells. Therefore, blowing a gas into the broth without foaming would otherwise be almost impossible.

Besides an outer tubular membrane for microfiltration, an inner tubular membrane for ultrafiltration may be disposed in the outer tubular membrane to form a two-layered membrane having a gap between the membranes. The pressure at the inner surface of the ultrafiltration membrane is kept the same as or near to the pressure at the inner surface of the microfiltration membrane, in order to extract a low molecular weight filtrate only when the pressure at the ultrafiltration membrane is reduced. The low molecular weight filtrate and a filtrate containing a polymer can be separated from one another, when pressures in the ultrafiltration membrane and the microfiltration membrane are reduced. For back-wash, a low molecular weight nutrient solution is supplied under pressure to only the inner surface of the ultrafiltration membrane, or to the inner surfaces of both membranes. According to this arrangement, low molecular weight waste components can be altogether removed by a single filtration apparatus and back-wash of both films can be made simultaneously. Furthermore, it is possible to recover and re-utilize serum and growth factors which are excreted from cells. If a gap is defined between the two membranes, the microfiltration membrane can be back-washed and regenerated simultaneously and effectively from the inner side.

Any drastic increase of the cell concentration in the filtration apparatus is restricted by filtering a culture broth while flowing into the filtration apparatus, and then the concentrated suspension of the cells generated by back-wash after filtration is sent back to the culture tank.

Also, if the bottom of the filtration apparatus and the culture tank are connected by a piping arrangement, and a gas is blown into the filtration apparatus from a sparger disposed at the bottom of the filtration apparatus, it may be possible not only to prevent adhesion of the cells to the membrane surface and their propagation on the membrane surface and inside the membrane matrix but also to return the suspension in the filtration apparatus into the culture tank by a gas lift effect and to introduce the culture broth in the culture tank into the filtration apparatus. Accordingly, it is possible to mitigate the rise of cell concentration at the time of filtration, and to extend the life of the filtration membrane as well as to return to the culture tank the cell aggregate occurring at the time of film filtration to the culture tank. In this case, the gas that is introduced into the filtration apparatus passes up through the bottom of the culture tank and forms bubbles on the liquid surface in the culture tank. Therefore, the defoaming layer (of the same type as mentioned above for the filtration apparatus) is disposed on the liquid surface in the culture tank. Besides the measures described above, two piping arrangements for connecting the filtration apparatus and the culturing apparatus are prepared. One is used for transferring culture broth from the culture tank to the filtration apparatus at the time of filtration. A valve and a pump are fitted into the other piping arrangement, whereby culture broth in the culture tank is transferred to the bottom of the filtration apparatus at a linear speed of at least 2 cm/sec and in a volume at least 50% of the charged solution quantity of the filtration apparatus at the time of back-wash of the membrane or after the back-wash. The concentrated suspension of cells occurring at the time of back-wash is returned to the culture tank.

The living cells to which the present invention may be applied are not particularly limited, and include animal cells, plant cells and microorganism cells. The animal cells include, for example, various cells of vertebrates and invertebrates. The cells need not be only single cells but may also be cell aggregates.

Plant cells include the cells and cell aggregates of higher plants, and the cells and cell aggregates of algae.

The culturing method and the culturing apparatus for the method are not particularly limited, either, but are primarily suitable for the cells under the suspended state. In the case of the suspension culture wherein cells are supported on carriers such as microbeads and gell particles for immobilization of cells, there are many cells that fall off from the carrier particles and the cells are suspended in the solution. Accordingly, these culturing methods, conditions, culturing apparatuses (culture tanks), etc., are selected in accordance with the cells used, the object of the culture, the scale and the control method. Particularly, the temperature,

EP 0 371 783 B1

pH, the dissolved oxygen concentration, the carbonic acid concentration, the oxidation-reduction potential, etc., are controlled suitably in accordance with the intended object.

Membranes having a pore diameter capable of separating the intended cells are suitably selected. The pore diameter is preferably from 5 $\mu$m to 0.5 $\mu$m. Known materials such as acetyl cellulose or poly-tetrafluoroethylene may suitably be used as the membrane material. Though the shape of the membrane may be selected for suitability, a hollow fiber module is an example suitable to increase the surface area of the membrane and to provide a liquid transfer port. Filtrate can be extracted conveniently by gathering both ends of a plurality of hollow fibers, by connecting both ends to form a loop and extracting the filtrate from a suitable position of the loop, or by closing one of the ends and gathering the other end of each hollow fiber so as to extract the filtrate from the bundled tubes.

Filtration under reduced pressure is ordinarily used as the filtration system. If filtration under elevated pressure is used, it must take into account the drop of pH due to carbon dioxide gas dissolving in the solution, and damage of brittle cells. The direction of the membrane surface inside the filtration apparatus is not particularly limited, and its arrangement and structure are selected so that the bubbles come efficiently into contact with the membrane surface. For example, the direction of the hollow fibers can be disposed either vertically or horizontally. The gas flowing method is not particularly limited either,but a flow of at least 0.1 m$\ell$/min per cm$^2$ surface area of membrane is necessary. The diameter of bubbles is selected suitably to be 1 to 10 mm, for example. The gas to be blown is selected suitably from among those which are not toxic to the cells. Examples include nitrogen, argon and air. The filtration pressure is selected suitably in accordance with the material, the solution, the cell concentration, the operating conditions, and the like, and is suitably from 0.01 ~ 5 kg/cm$^2$.

Defoaming of the foams on the liquid surface is made by the defoaming layer disposed on the surface of the liquid. The defoaming layer has a large number of openings capable of passing the gas. Its open ratio is preferably at least 50% and the diameter of the openings is preferably from 2 to 50 mm. At least the surface of the defoaming layer is made of a hydrophobic material which forms a contact angle of at least 80° with the culture solution or the liquid of the medium. A suitable example is an organic polymer of silane or siloxane having a carbon number of at least 10. Though the defoaming layer may be used as a single layer or a plurality of layers, the latter is preferable in consideration of the drop of water repellency of the hydrophobic surface due to the deposition of the cells for the operation over a long period. The drop of water repellency of the defoaming layer can be recovered by washing its surface with the culture filtrate, the medium or the culture broth.

As described above, the culture broth in the culturing apparatus is withdrawn outside the apparatus, the gas is brought into contact with the filtration membrane immersed in the culture broth thus withdrawn and filtration under reduced pressure is carried out to separate the cells and to return them into the culturing apparatus. If filtration is conducted without the contact of the gas, the cells that enter the membrane matrix and adhere thereto gradually propagate and clog the pores of the membrane. This clogging proceeds irreversibly even if back-wash is conducted using the liquid culture medium or the like as the washing solution. The pressure drop on filtration increases drastically, and ultimately filtration becomes impossible. Animal cells are likely to adhere to the membrane or to other cells, leading to clogging of the membrane. In contrast, if filtration is carried out while the gas is brought into contact with the membrane, adhesion of the cells to the membrane surface and the propagation of the cells on the surface and the matrix can be completely prevented so that clogging of the membrane can be prevented. Accordingly, if filtration and back-wash are cyclicly carried out regeneration and use of the membrane can be made for a period as long as one month or more.

Next, the present invention will be described in detail with reference to embodiments thereof.

Example 1:

Perfusion culture of animal cells was carried out by use of a culturing system shown in Fig. 1.

Prior to the culture, steam was supplied to each tank of the system and to each piping arrangement to heat them at 125°C for at least 30 minutes for sterilization. A medium having the following composition was charged under microorganism-free conditions into a medium tank 11.

4

| Eagle MEM medium (a product of Nissui Seiyaku K.K., Eagle MEM Nissui ①) | 9.4 g/ℓ |
| glutamine | 0.92 g/ℓ |
| 7.5% aqueous solution of sodium hydrocarbonate | 29 mℓ/ℓ |
| glucose | 20 g/ℓ |
| bovine serum | 100 mℓ/ℓ |

Rat liver cancer cell JTC-1 (Japan Tissue Culture No. 1) was used as a seed cell. A suspension of the seed cells used for the culture was prepared in the following way.

Five milliliters of the medium described above was charged into each of 15 flat flasks having a capacity of 20 mℓ and the JTC-1 strain described above was inoculated and subjected to setting culture in a concentration of $1 \times 10^5$ cells/mℓ. After being cultured for three days, the cells adhering to the inner surface of the flask were peeled off and joined with the cells suspended in the solution to obtain 75 mℓ of the culture solution having a cell concentration of $4.0 \times 10^5$ cells/mℓ. This culture solution was charged under microorganism-free conditions and dividedly into a centrifugal tube which had been treated under microorganism-free conditions, and the cells were recovered by suspension type open centrifuge. The cells were inoculated to two 1-ℓ roller bottles containing 75 mℓ of the medium and cultured at 37°C for three days. This operation was repeated several times to increase the quantity of the culture broth to obtain 2 ℓ of culture broth having a concentration of $1.0 \times 10^6$ cells/mℓ. The cells are recovered from this culture broth in the manner described above and suspended in 500 mℓ of the medium to obtain the seed cell suspension.

In the culturing system, a membrane filtration-apparatus 6, culturing vessel 1, a medium tank 11 and a culture filtrate tank 13 are connected by liquid transfer piping arrangements 24, 25, 26 and 29. A valve and a liquid transfer pump on each liquid transfer piping arrangement are connected to a process sequencer 10.

The culture tank has an effective capacity of 5 ℓ and is made of a stainless steel. Temperature, pH and DO as well as a stirrer 3 are automatically controlled. Oxygen is supplied in an overlay method to the liquid surface of the culture tank from a compressor 4 by an air transfer piping arrangement 28 through an air filter 5. Oxygen is dissolved by diffusion from the liquid surface and supplies dissolved oxygen necessary for the propagation of the cells.

After 4.5 ℓ of the medium, which was the same as used in seed culture, was introduced under microorganism-free conditions into the culturing apparatus that was steam-treated, 0.5 ℓ of the seed cell suspension described above was inoculated to the medium to adjust the cell concentration to $4.1 \times 10^5$ cells/mℓ. While microbial free air was blown at a rate of $10 \sim 30$ ℓ/min from the side wall of the tank onto the liquid surface, culture was carried out at a stirring speed of 20 r.p.m., 37°C and pH of $7.0 \sim 7.6$. The flow-rate of air was adjusted automatically so that the dissolved oxygen concentration (DO) in the culture solution became 1 ppm.

One liter of the culture broth 2 was transferred to the filtration apparatus 6 by opening the valve 20 and operating the pump 16. The filtration apparatus 6 has an effective volume of 1.3 ℓ and is made of stainless steel. A hollow fiber module of a microfiltration membrane 7 is disposed in the culture broth. Five hollow fiber membranes, which are made of tetrafluoroethylene, have an outer diameter of 2 mm, an inner diameter of 1 mm and a pore diameter of 0.8 μm and are 1 m long, are juxtaposed and both terminals are gathered to a common piping arrangement. Furthermore, both piping arrangements are connected to the medium tank 11 by a medium transfer piping arrangement 25 and to the culture filtrate tank 13 by a culture filtrate transfer piping arrangement 26. A sparger 9 (diameter 30 mm; ring sparger; 10 holes of diameter 1 mm) is disposed at the bottom of the filtration apparatus 6 so that bubbles of nitrogen gas can be brought into contact with the microfiltration membrane 7 from a pressure vessel 15 through a gas transfer piping arrangement 27. A defoaming layer 8 is disposed on the liquid surface. The defoaming layer 8 is a stainless steel net (one side of mesh: 3 mm) having a rectangular mesh with a thin coat of a silane-type organic polymer (viscosity: $1 \times 10^5$ cps) on its surface. A piping arrangement 29 is disposed at the bottom of the tank in order to return the cell suspension obtained after filtration and back-wash into the culture vessel 1.

Nitrogen gas was ventilated from the sparger towards the microfiltration membrane at a rate of 100 mℓ/min in the culture broth 2 introduced into the filtration apparatus 6 and the resulting foams were broken up by the defoaming layer. Filtrate 14 was withdrawn by the pump 19 and transferred to the filtrate storage tank 13. The filtration speed was set to 0.5 ℓ/h and filtration was stopped when the filtrate quantity reached 0.5 ℓ.

Next, 0.5 ℓ of a fresh medium 12 in the medium tank 11 was pressure-fed as a back-wash solution into the microfiltration membrane 7 at a rate of 3 ℓ/h through the piping arrangement 25. The resulting cell suspension was returned to the culturing vessel 1 by the pump 17 through the piping arrangement 29. This process was carried out 10 cycles/day and the culture was continued for 30 days. The viable cell concentration and the survival ratio in the culturing vessel 1 and the pressure loss at the time of filtration in this instance are tabulated in Table 1 below.

Since the survival ratio (viable cells/total cells × 100) of the cells during the culture was as high as 90% or more and the pressure drop at membrane filtration did not increase, it can be understood that the present system cultured the cells efficiently without clogging the membrane.

## Table 1

| Item \ Days of culture (days) | 0 | 1 | 2 | 5 | 10 | 20 | 30 |
|---|---|---|---|---|---|---|---|
| Viable cell concentration ($\times 10^5$ cells/m$\ell$) | 4.1 | 4.1 | 4.6 | 12 | 36 | 70 | 75 |
| Survival ratio (%) | 90 | 91 | 91 | 92 | 91 | 92 | 91 |
| Pressure drop at time of filtration (kg/cm$^2$) | 0.05 | 0.05 | 0.05 | 0.05 | 0.06 | 0.05 | 0.06 |

Example 2:

Perfusion culture of animal cells was carried out by use of a culturing system in which medium and animal cells of the same kind as those of Example 1 were used. A cell suspension prepared by centrifuging 2 $\ell$ of a culture broth of $1.0 \times 10^6$ cells/m$\ell$ and suspending the cells in the medium in the same way as in Example 1 was used as the seed cell suspension.

In this culture, a medium spray nozzle for washing the defoaming layer 8 is added to the upper part of the defoaming layer of the filtration-apparatus 6 of the system used in Example 1. The spray nozzle is the same type as the sparger 9.

While the same quantity of the medium as the quantity of filtrate was used for back-wash in Example 1, 480 m$\ell$ for back-wash was used in this example. The medium was also used for washing the defoaming layer (400m$\ell$/min, 0.5 min.) once a day.

The culture was carried out in the same way and under the same operation condition as those of Example 1. The result is tabulated in Table 2. The cells exhibited substantially the same type of propagation with a high survival ratio as in Example 1.

Table 2

| Item ＼ Days of culture (days) | 0 | 1 | 2 | 5 | 10 | 20 | 30 |
|---|---|---|---|---|---|---|---|
| Viable cell concentratin ($\times$ $10^5$ cells/m$\ell$) | 4.1 | 4.1 | 4.6 | 13 | 35 | 72 | 76 |
| Survival ratio (%) | 91 | 91 | 92 | 92 | 91 | 92 | 91 |
| Pressure drop at time of filtration (kg/cm$^2$) | -0.05 | -0.05 | -0.05 | -0.05 | -0.05 | -0.06 | -0.05 |

Example 3:

Perfusion culture of animal cells was carried out by use of a culturing system in which the same medium and the animal cells of the same strain as those of Example 1 were used. As the seed cell suspension, 2 $\ell$ of culture broth having a concentration of $1.0 \times 10^6$ cells/m$\ell$, which was prepared under the same operation condition as in Example 1, was centrifuged so as to separate the cells. A seed cell suspension was prepared by suspending the resulting cells in the medium.

The culturing apparatus was the same as the system of Example 1 except in that one end of each hollow fiber of the microfiltration membrane 7 in the filtration-apparatus 6 was closed, and the other ends of the hollow fibers were communicated and connected to the piping arrangement. The culture was carried out in the same way and under the same condition as in Example 1. The results are tabulated in Table 3 below. Substantially the same propagation was observed, with a high survival ratio, as in Example 1.

Table 3

| Days of culture (days) Item | 0 | 1 | 2 | 5 | 10 | 20 | 30 |
|---|---|---|---|---|---|---|---|
| Viable cell concentration ($\times 10^5$ cells/m$\ell$) | 3.9 | 4.1 | 4.7 | 14 | 35 | 75 | 79 |
| Survival ratio (%) | 91 | 91 | 91 | 92 | 92 | 92 | 92 |
| Pressure drop at time of filtration (kg/cm$^2$) | -0.05 | -0.05 | -0.05 | -0.05 | -0.05 | -0.05 | -0.05 |

Example 4:

Perfusion culture of animal cells was carried out by a culturing system in which the same medium and the animal cells of the same strain as those of Example 1 were used. However, serum was added to 4.5 $\ell$ of the medium at the start of the culture to a 10% concentration and no addition was made to the medium after the culture was started. A seed cell suspension was prepared by centrifuging 2 l of the culture broth having a concentration of $1.0 \times 10^6$ cells/ml, that was prepared under the same operation condition as in Example 1, and suspending the cells thus separated in the medium.

In this culturing system, the filtration membrane of the filtration apparatus 6 was a double-layer hollow fibre membrane formed by disposing ultrafiltration membranes (not shown) inside the microfiltration membranes with a gap between the membranes. During filtration, the pressure of the inner surface of the ultrafiltration membrane was kept at a level lower than that of the inner surface of the microfiltration membrane so that low molecular weight filtrate could be withdrawn from inside the ultrafiltration membrane, and a filtrate containing polymers was withdrawn separately from between the microfiltration membrane 7 and the ultrafiltration membrane. The low molecular weight filtrate was sent back to the low molecular weight filtrate tank 13 while the polymer-containing filtrate was sent back by a pump (not shown) to the culturing vessel 1 through a piping arrangement connected to the pump. The microfiltration membrane had the following structure: a hollow fibre made of the same material as that of Example 1 and having an outer diameter of 3 mm, an inner diameter of 2 mm and the same length was used. A hollow fiber membrane for ultrafiltration (pore diameter: 6,000 Dalton) made of acetyl cellulose and having an outer diameter of 1 mm and an inner diameter of 0.5 mm was inserted into the hollow fiber described above to form a hollow double-layered tube. Both ends of the films were gathered to the same withdrawal ports so as to withdraw both filtrates separately. The withdrawing rate of the low molecular weight filtrate was set to 0.45 $\ell$/h while the return rate of the polymer-containing filtrate was set to 0.05 $\ell$/h. When the sum of both filtrates reached 0.5 $\ell$, filtration was stopped. The culture was continued for 30 days in exactly the same way as in Example 1. Table 4 shows the living cell concentration, the survival ratio and the pressure loss at the time of filtration.

A high cell concentration was reached while maintaining a high survival ratio in the same way as in Examples Nos. 1 through 3. The viable cell concentration was higher than in the foregoing Examples, because polymeric growth factors excreted by the cells were recovered.

Table 4

| Item \ Days of culture (days) | 0 | 1 | 2 | 5 | 10 | 20 | 30 |
|---|---|---|---|---|---|---|---|
| Viable cell concentration ($\times 10^5$ cells/ml) | 4.0 | 4.3 | 4.9 | 18 | 45 | 92 | 110 |
| Survival ratio (%) | 91 | 91 | 91 | 92 | 92 | 92 | 92 |
| Pressure drop* at time of filtration (kg/cm²) | -0.07 | -0.07 | -0.07 | -0.07 | -0.06 | -0.06 | -0.08 |

*: pressure difference between inner pressure of ultrafiltration film and outer pressure of microfiltration film

Comparative Example 1:

Culture of the JTC-1 strain was carried out by use of a culturing system in which a seed cell suspension was prepared in the same way as in Example 1, and the medium had the same composition as that of Example 1.

This culturing system was produced by removing the sparger 9 and the defoaming layer 8 of the filtration-separation apparatus of the system of Example 1 and by adding a stirring blade 3 for stirring the suspension. The operation method was otherwise the same as that of the Examples of the invention.

The results are tabulated in Table 5. In comparison with Examples 1 ~ 4, clogging occurred more readily, pressure loss increased greatly, and the culture was stopped on the 20th day. Both the viable cell concentration and survival ratio were smaller than those of the Examples, owing to cell aggregation at the membranes.

Table 5

| Days of culture (days) Item | 0 | 1 | 2 | 5 | 10 | 20* |
|---|---|---|---|---|---|---|
| Viable cell concentration (x $10^5$ cells/m$\ell$) | 4.1 | 4.2 | 4.2 | 7 | 10 | 13 |
| Survival ratio (%) | 91 | 90 | 85 | 80 | 71 | 70 |
| Pressure drop at time of filtration (kg/cm$^2$) | 0.05 | 0.1 | 0.3 | 0.5 | 1.2 | 1.6 |

*: stop of culture

Example 5:

Perfusion cell of animal cells was carried out by use of the culturing system shown in Fig. 2. This system was different from the culturing system of Fig. 1 used in Example 1 in the following points.

1) The filtration apparatus was disposed underneath the culture tank. The upper part of the filtration apparatus communicated into the tank bottom by a transfer piping arrangement 40 so that culture broth in the filtration apparatus could be sent back to the culture tank by a gas lift effect from gas sparging at the bottom of the filtration apparatus. The volume of the filtration apparatus was the same, but its upper and lower end portions were tapered so as to effect the rising flow of the solution smoothly.

2) No defoaming layer was disposed in the filtration apparatus, but a defoaming layer of the same kind as before was disposed on the liquid surface in the culture tank.

3) The supply of oxygen to the culture tank was changed from the overlay system of Fig. 1, to a system ventilating the gas into the broth from a sparger disposed at the bottom of the tank.

The culturing system of Fig. 2 was steam-sterilized in the same way as in Example 1 and 4.5 $\ell$ of the medium of the same kind was introduced into the culture tank and the filtration apparatus. Next, 0.5 $\ell$ of the seed cell suspension prepared in the same way as in Example 1 was inoculated to fresh medium to make the cell concentration to 3.9 $\times 10^5$ cells/m$\ell$. Microbe-free air was a sparged at a flow rate of 10 ~ 30 $\ell$/min from the sparger at the bottom of the culture tank and while the bubbles formed on the liquid surface were ruptured by the defoaming layer, culture was carried out at a stirring speed of 20 r.p.m., at 37°C and pH of 7.0 ~ 7.6. The flow -rate was adjusted so that the dissolved oxygen concentration (DO) in the culture broth reached 1 ppm.

During the filtration operation, the valve 20 was opened and a circulating flow was formed by the rise of the gas from the sparger 9 disposed below the filtration apparatus. The back-wash of the membrane was made 10 cycles per day in the same way as in Example 1 and the culture was continued for 31 days. In this instance, the living cell concentration and the survival ratio in the culturing apparatus 1 were lowered, and considerable pressure drop at the time of filtration took place. The drop occurred because during the

EP 0 371 783 B1

filtration, the culture broth was always supplied and circulated from the culture tank to the filtration tank by the contact of bubbles with the membrane and by the gas lift.

Table 6

| Days of culture (days) Item | 0 | 1 | 2 | 5 | 10 | 20 | 30 |
|---|---|---|---|---|---|---|---|
| Viable cell concentration ($\times$ 10$^5$ cells/m$\ell$) | 4.1 | 4.1 | 4.1 | 15 | 40 | 85 | 98 |
| Survival ratio (%) | 90 | 91 | 91 | 91 | 91 | 91 | 91 |
| Pressure drop at time of filtration (kg/cm$^2$) | -0.03 | -0.03 | -0.03 | -0.04 | -0.04 | -0.04 | -0.04 |

Example 6:

Perfusion culture of animal cells was carried out by use of the culturing system. In this system, a liquid transfer piping arrangement corresponding to a bypass was added to the culturing system of Example 5 besides the liquid transfer pipe for connecting the culture tank 1 and the bottom of the filtration apparatus 6. A valve and a pump were disposed in this piping arrangement and they were used for sending the concentrated cell suspension in the filtration apparatus positively back into the culture tank after back-wash. In this instance, the rate of returning the cell suspension to the culture tank was at a rate of 250 m$\ell$/min for 2 minutes. The process of filtration and back-wash was carried out regularly at 10 cycles per day, in the same way as in Example 1, and culture was continued for 30 days. Table 7 shows the living cell concentration and survival ratio inside the culturing vessel 1 in this instance and the pressure loss at the time of filtration.

11

Table 7

| Days of culture (days) Item | 0 | 1 | 2 | 5 | 10 | 20 | 30 |
|---|---|---|---|---|---|---|---|
| Viable cell concentration ($\times 10^5$ cells/m$\ell$) | 4.1 | 4.1 | 4.1 | 13 | 42 | 80 | 82 |
| Survival ratio (%) | 90 | 90 | 92 | 91 | 92 | 91 | 91 |
| Pressure drop at time of filtration (kg/cm$^2$) | ⊢0.04 | ⊢0.04 | ⊢0.04 | ⊢0.04 | ⊢0.04 | ⊢0.05 | ⊢0.05 |

**Claims**

1. A method of culturing living cells wherein cells are cultured in a cell culture broth (2) in a culture vessel (1); the method comprising:
   withdrawing cell culture broth (2) from the culture vessel (1) to a filtration device (6) having a microfiltration membrane (7);
   microfiltering the cell culture broth (2) through the microfiltration membrane (7), while contacting the immersed surface of the membrane (7) with a gas which is non-toxic to the cells;
   using a hydrophobic defoaming layer (8) at the liquid surface to rupture bubbles and thereby inhibit the formation of foam;
   returning cells to the culture vessel (1) after the microfiltration, and withdrawing filtrate (14) from the culture.

2. A method according to claim 1 in which the membrane (7) is back-washed with a liquid medium (12) to carry the cells back to the culture vessel (1).

3. A method according to claim 1 or claim 2 in which the withdrawal of cell culture broth (2) from the culture vessel (1) to the filtration device (6) is intermittent.

4. A method according to any one of claims 1 to 3 in which the microfiltration membrane (7) and the non-toxic gas are contacted by bubbling the non-toxic gas onto the membrane (7) in the filtration device (6).

5. A method according to any one of the preceding claims in which the non-toxic gas is nitrogen, argon or air.

6. A method according to any one of the preceding claims in which the microfiltration membrane (7) is tubular and has a tubular ultrafiltration membrane nested within it at a spacing, whereby separate filtrates containing respectively polymeric and low molecular weight species are obtainable.

12

7.  A method according to claim 6 in which said filtrate of polymeric species is reintroduced into the culture.

8.  A method according to any one of the preceding claims in which the volume of culture broth (2) withdrawn to the filtration device (6) is at least 50% of the volume of a liquid medium (12) used for back-washing cells from the filtration membrane (7).

9.  Apparatus for cell culture, comprising;
    (a) a culture vessel (1) for holding a culture broth (2) in which cells are cultured in a suspended state;
    (b) a filtration device (6) comprising a microfiltration membrane (7);
    (c) means (16,24) for transferring culture broth from the culturing vessel (1) to the filtration device (6);
    (d) means (9) for blowing a gas into culture broth in the filtration device (6), to contact the microfiltration membrane (7);
    (e) means (8) for inhibiting foam formation on the culture broth surface;
    (f) means (29,30;40) for returning cells from the filtration device (6) to the culture vessel (1), and
    (g) means (19,26) for withdrawing filtrate (14) from the filtration device (6).

10. Apparatus according to claim 9 in which the microfiltration membrane (7) is an elongate tube.

11. Apparatus according to claim 9 or claim 10 in which the microfiltration membrane (7) is tubular and a tubular ultrafiltration membrane is disposed inside it with a gap between the membranes.

12. Apparatus according to any one of claims 9 to 11, comprising means for washing the foam inhibiting means (8).

13. Apparatus according to any one of claims 9 to 12, in which the gas-blowing means (9) comprise a sparger beneath the microfiltration membrane (7).

14. Apparatus according to any one of claims 9 to 13 in which the filtration device (6) is positioned below the culturing vessel (1) whereby gas bubbles rise in use from the filtration device (6) into the culturing vessel (1), and the foam-inhibiting means (8) is provided in the culturing vessel (1).

**Patentansprüche**

1.  Verfahren zum Züchten lebender Zellen, bei dem Zellen in einer Zellkulturbouillon (2) in einem Züchtungsgefäß (1) gezüchtet werden; welches Verfahren folgendes aufweist:
    - Entnehmen von Zellkulturbouillon (2) aus dem Züchtungsgefäß (1) in eine Filtriervorrichtung (6) mit einer Mikrofiltriermembran (7);
    - Mikrofiltern der Zellkulturbouillon (2) durch die Mikrofiltriermembran (7), während die eingetauchte Fläche der Membran (7) mit einem Gas in Berührung gebracht wird, das für die Zellen ungiftig ist;
    - Verwenden einer hydrophoben Entschäumungsschicht (8) an der Flüssigkeitsoberfläche, um Blasen zu zerreißen und dadurch das Ausbilden von Schaum zu verhindern; und
    - Rückführen von Zellen in das Züchtungsgefäß (1) nach der Mikrofiltrierung, und Entnehmen von Filtrat (14) aus der Kultur.

2.  Verfahren nach Anspruch 1, bei dem die Membran (7) mit einem flüssigen Medium (12) rückgewaschen wird, um die Zellen in das Züchtungsgefäß (1) rückzuführen.

3.  Verfahren nach Anspruch 1 oder Anspruch 2, bei dem das Entnehmen von Zellkulturbouillon (2) aus dem Züchtungsgefäß (1) zur Filtriervorrichtung (6) intermittierend ist.

4.  Verfahren nach einem der Ansprüche 1 bis 3, bei dem die Mikrofiltriermembran (7) und das ungiftige Gas dadurch in Berührung gebracht werden, daß das ungiftige Gas blasenförmig auf die Membran (7) in der Filtriervorrichtung (6) aufgebracht wird.

**5.** Verfahren nach einem der vorstehenden Ansprüche, bei dem das nichtgiftige Gas Stickstoff, Argon oder Luft ist.

**6.** Verfahren nach einem der vorstehenden Ansprüche, bei dem die Mikrofiltriermembran (7) rohrförmig ist und eine darin unter Einhaltung eines Abstandes eingebettete rohrförmige Ultrafiltriermembran aufweist, wodurch getrennte Filtrate, die polymere Teilchen bzw. solche mit niedrigem Molekulargewicht enthalten, erhalten werden können.

**7.** Verfahren nach Anspruch 6, bei dem das Filtrat polymerer Teilchen in die Kultur rückgeführt wird.

**8.** Verfahren nach einem der vorstehenden Ansprüche, bei dem das Volumen der der Filtriervorrichtung (6) entnommenen Kulturbouillon (2) mindestens 50 % des Volumens eines flüssigen Mediums (12) ausmacht, das zum Rückwaschen von Zellen aus der Filtriermembran (7) verwendet wird.

**9.** Vorrichtung zur Zellzüchtung mit:
(a) einem Züchtungsgefäß (1) zum Aufnehmen einer Kulturbouillon (2), in der Zellen in suspendiertem Zustand gezüchtet werden;
(b) einer Filtriervorrichtung (6) mit einer Mikrofiltriermembran (7);
(c) einer Einrichtung (16, 24) zum Übertragen von Kulturbouillon vom Züchtungsgefäß (1) in die Filtriervorrichtung (6);
(d) einer Einrichtung (9) zum Einblasen eines Gases in die Kulturbouillon in der Filtriervorrichtung (6), um es in Berührung mit der Mikrofiltriermembran (7) zu bringen;
(e) einer Einrichtung (8) zum Verhindern von Schaumbildung auf der Oberfläche der Kulturbouillon;
(f) einer Einrichtung (29, 30; 40) zum Rückführen von Zellen von der Filtriervorrichtung (6) in das Züchtungsgefäß (1); und
(g) einer Einrichtung (19, 26) zum Entnehmen von Filtrat (14) aus der Filtriervorrichtung (6).

**10.** Vorrichtung nach Anspruch 9, bei der die Mikrofiltriermembran (7) ein langgestrecktes Rohr ist.

**11.** Vorrichtung nach Anspruch 9 oder Anspruch 10, bei der die Mikrofiltriermembran (7) rohrförmig ist und eine rohrförmige Ultrafiltriermembran in ihr unter Einhaltung eines Spaltes zwischen den Membranen angeordnet ist.

**12.** Vorrichtung nach einem der Ansprüche 9 bis 11, mit einer Einrichtung zum Waschen der Schaumverhinderungseinrichtung (8).

**13.** Vorrichtung nach einem der Ansprüche 9 bis 12, bei der die Gaseinblaseinrichtung (9) eine Verteilereinrichtung unter der Mikrofiltriermembran (7) aufweist.

**14.** Vorrichtung nach einem der Ansprüche 9 bis 13, bei der die Filtriervorrichtung (6) unter dem Züchtungsgefäß (1) angeordnet ist, wodurch Gasblasen bei Benutzung von der Filtriervorrichtung (6) in das Züchtungsgefäß (1) hochsteigen und die Schaumverhinderungseinrichtung (8) im Züchtungsgefäß (1) angeordnet ist.

**Revendications**

**1.** Procédé de culture de cellules vivantes dans lequel les cellules sont cultivées dans un bouillon de culture de cellules (2) dans un récipient de culture (1); le procédé comprenant :
le soutirement du bouillon de culture de cellules (2) du récipient de culture (1) vers un dispositif de filtration (6) ayant une membrane de microfiltration (7);
la microfiltration du bouillon de culture de cellules (2) à travers la membrane de microfiltration (7), tout en mettant en contact la surface immergée de la membrane (7) avec un gaz qui est non toxique pour les cellules;
l'utilisation d'une couche (8) hydrophobe enlevant la mousse sur la surface liquide pour rompre les bulles et par là inhiber la formation de mousse;
le retour des cellules dans le récipient de culture (1) après la microfiltration, et le soutirement du filtrat (14) de la culture.

14

**2.** Procédé selon la revendication 1, dans lequel la membrane (7) est lavée en retour avec un milieu liquide (12) pour emmener les cellules dans le récipient de culture (1).

**3.** Procédé selon la revendication 1 ou 2, dans lequel le soutirement du bouillon de culture de cellules (2) du récipient de culture (1) vers le dispositif de filtration (6) est intermittent.

**4.** Procédé selon l'une quelconque des revendications 1 à 3, dans lequel la membrane de microfiltration (7) et le gaz non toxique sont mis en contact par bullage du gaz non toxique sur la membrane (7) dans le dispositif de filtration (6).

**5.** Procédé selon l'une quelconque des revendications précédentes, dans lequel le gaz non toxique est l'azote, l'argon ou l'air.

**6.** Procédé selon l'une quelconque des revendications précédentes, dans lequel la membrane de microfiltration (7) est tubulaire et a une membrane d'ultrafiltration tubulaire nichée dans celle-ci sur un écartement, ce par quoi des filtrats séparés contenant respectivement des espèces polymèriques et des espèces de bas poids moléculaire peuvent être obtenues.

**7.** Procédé selon la revendication 6, dans lequel ledit filtrat des espèces polymèriques est réintroduit dans la culture.

**8.** Procédé selon l'une quelconque des revendications précédentes dans lequel le volume du bouillon de culture (2) soutiré vers le dispositif de filtration (6) est au moins 50 % du volume d'un milieu liquide (12) utilisé pour le lavage en retour des cellules de la membrane de filtration (7).

**9.** Appareil pour culture de cellules, comprenant :
(a) un récipient de culture (1) pour contenir un bouillon de culture (2) dans lequel des cellules sont cultivées dans un état suspendu ;
(b) un dispositif de filtration (6) comprenant une membrane de microfiltration (7);
(c) des moyens (16, 24) pour transférer le bouillon de culture du récipient de culture (1) au dispositif de filtration (6);
(d) des moyens (9) pour souffler un gaz: dans le bouillon de culture dans le dispositif de filtration (6), pour contacter la membrane de microfiltration (7);
(e) des moyens (8) pour inhiber la formation de mousse sur la surface du bouillon de culture;
(f) des moyens (29, 30 ; 40) pour renvoyer des cellules du dispositif de filtration (6) au récipient de culture (1), et
(g) des moyens (19, 26) pour soutirer le filtrat (14) du dispositif de filtration (6).

**10.** Appareil selon la revendication 9, dans lequel la membrane de microfiltration (7) est un tube élongué.

**11.** Appareil selon la revendication 9 ou 10, dans lequel la membrane de microfiltration (7) est tubulaire et une membrane d'ultrafiltration tubulaire est disposée à l'intérieur de celle-ci avec une distance entre les membranes.

**12.** Appareil selon l'une quelconque des revendications 9 à 11, comprenant des moyens pour laver les moyens (8) inhibants la mousse.

**13.** Appareil selon l'une quelconque des revendications 9 à 12, dans lequel les moyens de soufflage du gaz (9) comprennent un arroseur sous la membrane de microfiltration (7).

**14.** Appareil selon l'une quelconque des revendications 9 à 13, dans lequel le dispositif de filtration (6) est situé sous le récipient de culture (1) ce par quoi les bulles de gaz s'échappent en utilisation du dispositif de filtration (6) dans le récipient de culture (1), et les moyens d'inhibition de mousse (8) sont prévus dans le récipient de culture (1).

EP 0 371 783 B1

# FIG. 1

# FIG. 2